# EUROPÄISCHE PATENTSCHRIFT

(11) **EP 2 569 274 B1**
(45) Veröffentlichungstag und Bekanntmachung des Hinweises auf die Patenterteilung: **29.06.2016**
(21) Anmeldenummer: 11713266.2
(22) Anmeldetag: 07.04.2011
(51) Int. Cl.: C07C 51/44, C07C 51/46, C07C 57/04, B01D 3/14, B01D 3/36

(54) **DESTILLATITIVE ABTRENNUNG VON ACRYLSÄURE ÜBER EINEN SEITENABZUG**
DISTILLATIVE REMOVAL OF ACRYLIC ACID VIA A SIDE DRAW
SÉPARATION PAR DISTILLATION D'ACIDE ACRYLIQUE PAR L'INTERMÉDIAIRE D'UNE CONDUITE D'ÉVACUATION LATÉRALE

(30) Priorität: 10.05.2010 DE 102010028781
(43) Veröffentlichungstag der Anmeldung: 20.03.2013
(73) Patentinhaber: Evonik Degussa GmbH, 45128 Essen (DE)
(72) Erfinder: MEIER, Ralf, 44265 Dortmund (DE); MOSLER, Jürgen, 45772 Marl (DE)
(86) Internationale Anmeldenummer: PCT/EP2011/055429
(87) Internationale Veröffentlichungsnummer: WO 2011/141240

(56) Entgegenhaltungen:
- EP-A1- 2 085 376
- WO-A2-2010/107284
- DE-A1- 10 138 150
- DE-A1- 19 924 533
- US-A1- 2004 225 152
- US-A1- 2009 253 934

## Beschreibung

Die Erfindung betrifft ein Verfahren zur Abtrennung von Acrylsäure aus einer Zusammensetzung enthaltend Acrylsäure und zumindest ein Begleitprodukt mit den Merkmalen a) bis f) des Anspruch 1. Ein Verfahren dieser Gattung ist sowohl aus US5910607, als auch aus US7189872 bekannt.

Acrylsäure (Prop-2-ensäure) ist ein Grundbaustein für die Herstellung von vornehmlich in Hygieneprodukten eingesetzten Superabsorbern. Es wird im großtechnischen Maßstab durch katalytische Dampfphasenreaktion von Propen mit Sauerstoff erhalten.

Dabei entstehen neben der gewünschten Acrylsäure auch unerwünschte Begleitprodukte, die bei der Weiterverarbeitung der Acrylsäure hinderlich sind. Bei diesen Begleitprodukten handelt es sich im Wesentlichen um Essigsäure, Aldehyde und Wasser. Ein wichtiger Schritt bei der Herstellung von Acrylsäure besteht demnach darin, die aus der Dampfphasenreaktion stammende, acrylsäurehaltige Zusammensetzung von den unerwünschten Begleitprodukten zu reinigen, sodass reine Acrylsäure übrig bleibt. Die Erfindung befasst sich dieser Aufreinigung.

Da Acrylsäure stark zur Polymerisation neigt, ist ihr stets ein stabilisierender Inhibitor zuzusetzen. Bei dem in US7189872 beschriebenen gattungsbildenden Verfahren geschieht die destillative Aufarbeitung des mittels Schleppmittels entwässerten Sumpfproduktes mit Hilfe von drei hintereinander geschalteten Destillationskolonnen, welche nacheinander die leicht- und schwersiedenden Begleitprodukte aus dem Strom abscheiden. Aufgrund der großen Anzahl von Destillationskolonnen ist eine solche Anlage vergleichsweise kostspielig zu installieren und zu betreiben; der Energie- und Inhibitorverbrauch steigt mit der Anzahl der Kolonnen.

In der US5910607 wird lediglich die destillative Abscheidung der leicht siedenden Essigsäure aus dem entwässerten Sumpfprodukt thematisiert. Zur Abscheidung von schwer siedenden Begleitprodukten wie Aldehyden wird nichts näher ausgeführt.

Aus der EP2085376A1 ist ein Verfahren zur Aufreinigung vom Methacrylsäure bekannt. Ein Wesentlicher Unterschied gegenüber der Aufreinigung von Acrylsäure besteht darin, dass das Polymerisationspotenzial von monomerer Acrylsäure deutlich höher ist als von Methacrylsäure und dementsprechend für die Aufarbeitung von Acrylsäure niedrigere thermische Belastungen und damit niedrigere Prozesstemperaturen eingehalten werden müssen. Aus diesem Grunde ist die Aufreinigung von Methacrylsäure mit der von Acrylsäure kaum technisch vergleichbar.

DE19924533A1, DE10138150A1 und US2009/253934A1 offenbaren Verfahren zur destillativen Abtrennung von Acrylsäure durch Verwendung eines Seitenabzugs. Sie offenbaren jedoch keine azeotropische Destillation.

Die zum Prioritätszeitpunkt noch unveröffentlichte WO2010/107284A2 offenbart die Aufreinigung von Acrylsäure in einer Destillationskolonne mit einer vertikalen Trennwand.

In Hinblick auf diesen Stand der Technik liegt der vorliegenden Erfindung die Aufgabe zu Grunde, ein Verfahren der eingangs genannten Gattung so weiterzubilden, dass es bei der Aufreinigung des bereits entwässerten Sumpfproduktes mit einer möglichst geringen Anzahl von Destillationskolonnen auskommt und dennoch eine zuverlässige Abscheidung der schwer und leicht siedenden Begleitprodukte gestattet.

Gelöst wird diese Aufgabe dadurch, dass die destillative Auftrennung des Sumpfproduktes in einer mit einem Seitenabzug ausgestatten Destillationskolonne erfolgt, aus deren Sumpf die schwersiedende Bestandteile des Begleitproduktes, von deren Kopf die leicht siedende Bestandteile des Begleitproduktes und aus deren Seitenabzug die Acrylsäure abgezogen wird.

Gegenstand der Erfindung ist mithin ein Verfahren zur Abtrennung von Acrylsäure aus einer Zusammensetzung enthaltend Acrylsäure und zumindest ein Begleitprodukt,
a) bei welchem die Zusammensetzung zunächst mit einem wässrigen Flüssigkeitsstrom dergestalt kontaktiert wird, dass eine flüssige Lösung enthaltend Acrylsäure, Begleitprodukt und Wasser anfällt,
b) bei welchem der flüssigen Lösung ein Schleppmittel zugegeben wird, welches mit Wasser der flüssigen Lösung ein heterogenes Minimalazeotrop bildet,
c) bei welchem die flüssige Lösung destillativ aufgetrennt wird in ein Kopfprodukt enthaltend das Minimalazeotrop und in ein Sumpfprodukt enthaltend Acrylsäure und Begleitprodukt,
d) bei welchem das Kopfprodukt in Wasser und rückführbares Schleppmittel getrennt wird,
e) und bei welchem das Sumpfprodukt destillativ aufgetrennt wird in Acrylsäure und Begleitprodukt,
f) wobei die destillative Auftrennung des Sumpfproduktes in einer einen Seitenabzug aufweisenden Destillationskolonne erfolgt, aus deren Sumpf schwersiedende Bestandteile des Begleitproduktes, von deren Kopf leicht siedende Bestandteile des Begleitproduktes und aus deren Seitenabzug die Acrylsäure abgezogen wird, wobei die Acrylsäure flüssig aus dem Seitenabzug abgezogen wird und die Destillationskolonne als Trennwandkolonne mit einer sich vertikal durch die Kolonne ersteckenden Trennwand ausgeführt ist.

Die vorliegende Erfindung beruht unter anderem auf der Erkenntnis, dass sowohl leicht siedende, als auch schwer siedende Begleitprodukte aus dem entwässerten Sumpfprodukt in einer einzigen Destillationskolonne abgeschieden werden können, wenn diese Destillationskolonne mit einem Seitenabzug versehen ist, aus welchem die gereinigte Acrylsäure entnommen wird.

Verglichen mit US7189872, kann dank der Verwendung der Destillationskolonne mit Seitenabzug der für die Aufreinigung der Acrylsäure notwendige apparative Aufwand bei Beibehaltung der in diesem Stand der Technik erzielten Reinheit minimiert werden. Dies senkt nicht nur die Investitions- sondern auch die Betriebskosten.

Erfindungsgemäß wird die Acrylsäure flüssig aus dem Seitenabzug abgezogen.

Der Abzug von flüssiger Acrylsäure mit geringem Schwersiederanteil von der Seite der Destillationskolonne ist dann möglich, wenn die Destillationskolonne als Trennwandkolonne ausgeführt ist. Eine Trennwandkolonne ist eine Destillationskolonne, welche eine sich im Wesentlichen vertikal durch die Kolonne ersteckenden Trennwand aufweist. Eine derartige Trennwandkolonne ist in US2471134 beschrieben.

Es sind unterschiedliche Gestaltvarianten einer Trennwandkolonne geeignet, welches sich durch den Verlauf der Trennwand unterscheiden:
Zunächst kann sich die Trennwand nicht über die volle Höhe der Destillationskolonne erstrecken, sondern nur etwa von einem trennwandlosen Kopfbereich bis zu einem trennwandlosen Sumpfbereich. Dementsprechend ist in einem kopfnahen und in einem sumpfnahe Bereich ein freier Übertritt des Einsatzstoffes über den gesamten Querschnitt der Kolonne möglich. Der trennwandlose Kopfbereich erstreckt sich bevorzugt über die oberen 10 % der Kolonnenböden, der trennwandlose Sumpfbereich über die unteren 10 bis 20 % der Kolonnenböden. Zweck dieser Gestaltung ist, dass die Abtrennung von Leicht- und Schwersiedern durch den Einsatz von nur einem Kondensator und einem Verdampfer ermöglicht wird.

Eine im Kopf- und Sumpfbereich trennwandlose Kolonne kann vorteilhaft dahingehend weitergebildet werden, dass der auf der Seite des Seitenabzugs liegende Teil der Destillationskolonne zum trennwandlosen Kopfbereich hin mit einer Sperrwand verschlossen ist. Dies hat den Vorteil, dass Verunreinigungen, die sich auf der Zulaufseite oberhalb der Zulaufposition in der Kolonne befinden, nicht im oberen Bereich auf die Seitenstromseite übertreten können und die Produktreinheit im Seitenstrom schmälern.

In einer weiteren Ausführungsform wird auf einen trennwandlosen Sumpfbereich verzichtet, sodass sich die Trennwand von dem trennwandlosen Kopfbereich bis zum Boden des Sumpfes erstreckt. Diese Variante hat den Vorteil, dass Komponenten, die sich auf der Zulaufseite unterhalb des Zulaufs befinden nicht im unteren Bereich auf die Seitenstromsite übertreten können. Auf diese Weise wird eine höhere Produktreinheit erzielt.

Bei der Bemessung der Trennwände aller Ausführungsformen der Trennwandkolonne ist die Gestaltungsregel zu beachten, dass sich die Trennwand von einem Höhenniveau oberhalb des Niveaus des Seitenabzugs bis zu einem Höhenniveau unterhalb des Niveaus des in die Destillationskolonne eingeleiteten Sumpfproduktes erstreckt. Dies hat zur Folge, dass der Einlass für das in der Trennwandkolonne zu trennende Sumpfprodukt durch die Trennwand von dem Seitenabzug, also dem Auslass für die Acrylsäure, abgeschirmt ist, sodass ein direkter Stofffluss vom Einlass des Sumpfproduktes zum Auslass unterbunden ist. Diese Maßnahme ist die Vorraussetzung für eine gute Abscheideleistung.

Das erfindungsgemäße Verfahren eignet sich besonders zur Aufreinigung einer Zusammensetzung, die neben Acrylsäure als Begleitprodukte Essigsäure und/oder Wasser und/oder Aldehyde aufweist. Essigsäure ist ein leicht siedendes Begleitprodukt, welches aus dem Kopf der Destillationskolonne abgezogen wird, währenddessen es sich bei den Aldehyden um Schwersieder handelt, die aus dem Sumpf der den Seitenabzug aufweisenden Destillationskolonne abgezogen werden.

Als Schleppmittel eignet sich ein organisches Lösungsmittel mit einem Siedepunkt zwischen 80 und 130°C, wie beispielsweise Toluol oder Heptan oder Cyclohexan oder Methylcyclohexan.

Bevorzugt erfolgt die destillative Auftrennung der flüssigen Lösung in einer Entwässerungskolonne durch geeignete Einstellung von Heizleistung und Schleppmittelmenge dergestalt, dass das Sumpfprodukt nahezu wasserfrei ist und die Konzentration des Schleppmittels in dem Sumpfprodukt 1 bis 15, bevorzugt 8 bis 12 Gew.-% beträgt. "Nahezu wasserfrei" bedeutet in diesem Zusammenhang weniger als 0,5 Gew-%, besser 0,05 bis 0,3 Gew-%. Dieser Konzentrationsbereich ist durch einen besonders vorteilhaften Betrieb gekennzeichnet. Zum Einen werden aufgrund der relativ niedrigen Sumpftemperaturen geringe Polymerisationsraten beobachtet. Zum Anderen zeigt sich, dass unter diesen Verfahrensparametern unerwünschtes Polymer bevorzugt in dem leicht zugänglichen Sumpfbereich der Kolonne anfällt. Auf Grund der sehr guten Zugänglichkeit lässt sich die Kolonne bei Reinigungsabstellung mit geringem Zeit- und Kostenbedarf vom Polymer und von sonstigen Ablagerungen befreien und es ergibt sich eine optimierte Anlagenverfügbarkeit.Die Erfindung soll nun anhand von Ausführungsbeispielen näher erläutert werden. Hierfür zeigen:
- Figur 1:: Anlage zur Durchführung des Verfahrens, schematisch;
- Figur 2:: Ausführungsform einer Destillationskolonne mit Seitenabzug und ohne Trennwand (außerhalb der beanspruchten Erfindung);
- Figur 3:: Ausführungsform einer Destillationskolonne mit Seitenabzug und Trennwand zwischen trennwandlosen Kopf- und Sumpfbereich;
- Figur 4:: Ausführungsform einer Destillationskolonne mit Seitenabzug und Trennwand vom trennwandlosen Kopfbereich bis zum Boden des Sumpfes;
- Figur 5:: Ausführungsform einer Destillationskolonne mit Seitenabzug und Trennwand zwischen trennwandlosen Kopf- und Sumpfbereich, zusätzlich mit seitenabzugseitiger Sperrplatte.

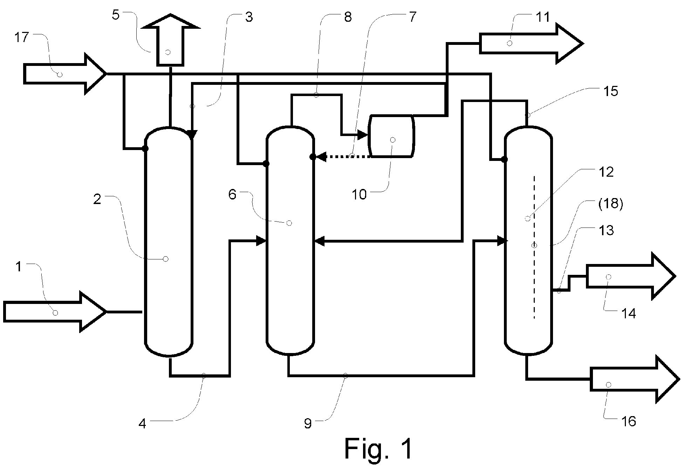

Eine Anlage zur Durchführung des erfindungsgemäßen Verfahrens ist schematisch in Figur 1 dargestellt. Ausgangsprodukt ist eine gasförmige, aus einem nicht dargestellten Reaktor stammende Zusammensetzung 1 enthaltend Acrylsäure und Begleitprodukt umfassend insbesondere Essigsäure, Wasser und Aldehyde. In einer Quenchkolonne 2 wird die Zusammensetzung 1 mit einem wässrigen Flüssigkeitsstrom 3 kontaktiert, wobei sich eine flüssige Lösung 4 von Wasser (20 bis 80 Gew-%), Acrylsäure (40-80 Gew-%) und verschiedenen kondensierbaren Begleitprodukten wie Essigsäure (1 bis 5 Gew-%) und Aldehyde ausbildet. Nichtkondensierbare Bestandteile der Zusammensetzung 1 verlassen die Quenchkolonne 2 am Kopf als Gasstrom 5.

Im nächsten Verfahrensschritt wird der wässrige Flüssigkeitsstrom 3 einer Entwässerungskolonne 6 zugeführt. Unter Zugabe eines Schleppmittels 7, welches mit dem Wasser ein heterogenes Minimalazeotrop bildet, wird das Wasser/Schleppmittelazeotrop 8 als Brüden am Kopf der Entwässerungskolonne 6 angetrennt. Die Entwässerungskolonne 6 weist vorzugsweise zwischen 5 und 20 theoretische Stufen auf. Der Kopfdruck der Entwässerungskolonne 6 beträgt zwischen 133 und 400 hPa, die Sumpftemperatur liegt unter 100 °C. Die Prozessvariablen Heizleistung und Schleppmittelmenge werden dabei so eingestellt, das sich ein nahezu wasserfreies Sumpfprodukt 9 (Wassergehalt kleiner als 0,5 Gew-%, vorzugsweise 0,05 bis 0,3 Gew-%) bei einer Schleppmittelkonzentration von 1 bis 15 Gew-%, vorzugsweise von 8 bis 12 Gew-% ergibt. Die Vorteile dieses Konzentrationsbereiches wurden bereits oben hervorgehoben.

Das gasförmige Wasser/Schleppmittelazeotrop 8 wird unter Ausbildung zweier flüssiger Phasen kondensiert (nicht dargestellt) und gelangt in einen Dekanter 10, in welchem die beiden Phasen Schleppmittel 7 und Wasser getrennt werden. Das Schleppmittel 7 wird in die Entwässerungskolonne 6 zurückgeführt, das Wasser gelangt teilweise als wässriger Flüssigkeitsstrom 3 zurück in die Quenchkolonne 2, ein Überschuss wird als Abwasser 11 ausgeschleust.

Das Sumpfprodukt 9 der Entwässerungskolonne 6, welches hauptsächlich aus Acrylsäure, Essigsäure und schwersiedenden Aldehyden besteht, wird zur weiteren Reinigung in eine Destillationskolonne 12 geführt, in welcher kombiniert eine weitere Leichtsiederabtrennung über Kopf und eine Schwersiederabtrennung über Sumpf erfolgt. Zu diesem Zwecke ist die Destillationskolonne 12 mit einem Seitenabzug 13 versehen, aus welchem die gereinigte Acrylsäure 14 dampfförmig oder flüssig entnommen wird. Betriebsparameter und Bauformen der Destillationskolonne 12 werden später erörtert. Die über Kopf aus der Destillationskolonne 15 abgezogenen, leicht siedenden Bestandteile 15 des Begleitproduktes wie Essigsäure werden in die Entwässerungskolonne 6 zurück geführt. Die schwer siedenden Bestandteile 16 des Begleitproduktes werden aus dem Sumpf der Destillationskolonne 12 ausgeschleust.

Im Gegensatz zu dem beschriebenen Stand der Technik wird bei dem erfindungsgemäßen Verfahren die Anzahl der Verfahrensschritte für die Reingewinnung von Acrylsäure 14 minimiert. Neben den geringen Investitionsaufwendungen ergeben sich auf Grund der geringeren Anzahl der Verfahrensschritte niedriger Energieverbrauche und auch ein geringere Bedarf an Polymerisationsinhibitoren 17, die jedem Destillationsschritt zugeführt werden müssen. Zusätzlich ergibt sich eine geringere Temperaturbelastung der monomeren Acrylsäure. Dies reduziert die Polymerisation der Acrylsäure und die Bildung von Acrylsäuredimeren, was wiederum die Produktausbeute steigert.

Außerdem führt die Aufteilung der Leichtsiederabscheidung in die Entwässerungskolonne 6 mit verbleibender Restkonzentration an Schleppmittel und die kombinierte Leicht/Schwersiederabtennung in der Destillationskolonne 12 mit Seitenabzug dazu, dass die unvermeidlich gebildeten polymeren Rückstände in dem sehr gut zugänglichen Sumpf der Entwässerungskolonne 6 anfallen. Eine Entfernung des Polymers und die Reinigung der Kolonne sind in diesem Bereich mit relativ geringem Aufwand möglich, sodass Stillstandszeiten der Anlage minimiert werden.

Gestaltung und Betriebsparameter der Destillationskolonne 12 mit Seitenabzug 13 sollen nun näher erläutert werden:
Die Destillationskolonne 12 sollte dreißig bis fünfundvierzig praktische Böden haben. Der Seitenabzug 13 sollte auf einem Höhenniveau liegen, welches 70 bis 80 % der gesamten Bodenzahl (von oben gezählt) entspricht. Der Kopfdruck sollte 40 bis 50 hPa betragen, die Kopftemperatur zwischen 35 und 50°C liegen. Die Sumpfverdampfung der Destillationskolonne 12 erfolgt über Umlaufverdampfer (Produkttemperatur kleiner 90° C) und nachfolgendem Dünnschichtverdampfer (Produkttemperatur kleiner 125 °C, bevorzugt 115 °C).

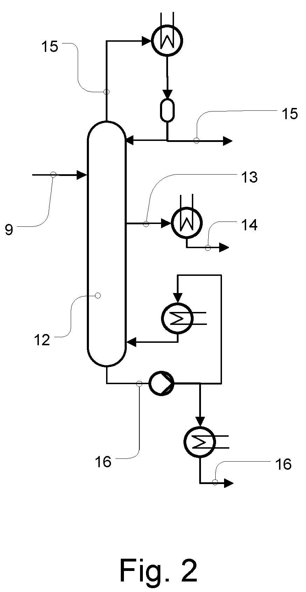

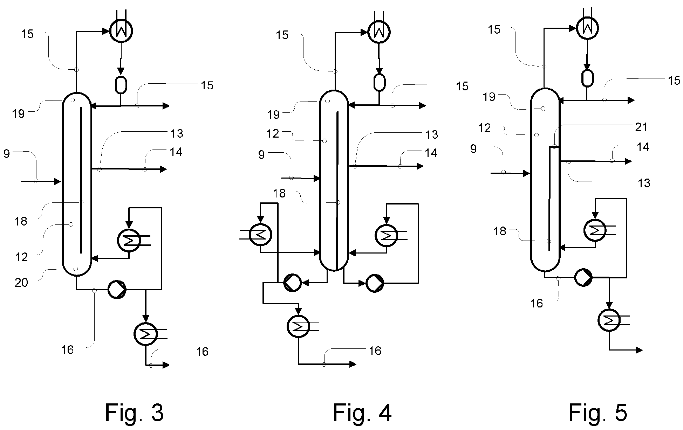

Wird die Acrylsäure 14 gasförmig abgezogen, kann eine trennwandlose Kolonne verwendet werden, wie in Figur 2 dargestellt (außerhalb der beanspruchten Erfindung). Der Sumpfstrom 9 wird dann auf einem Höhenniveau oberhalb des Seitenabzugs 13 in die Destillationskolonne 12 gefahren. Ein Abzug der Acrylsäure 14 im flüssigen Zustand erfordert eine mit einer Trennwand 18 ausgerüstete Destillationskolonne 12; vgl. Figuren 3, 4 und 5. Bei einer Trennwandkolonne wird der Sumpfstrom 9 auf einem Höhenniveau unterhalb des Höhenniveaus des Seitenabzugs 13 in die Destillationskolonne 12 gefahren. Die Trennwand 18 ist dabei stets so zu bemessen, dass sie sich von sich von einem Höhenniveau oberhalb des Niveaus des Seitenabzugs bis zu einem Höhenniveau unterhalb des Niveaus des in die Destillationskolonne 12 eingeleiteten Sumpfproduktes 9 erstreckt. Die oberen 10% der Böden bilden einen trennwandlosen Kopfbereich 19, unterhalb dessen der Verlauf der Trennwand 18 beginnt.

Die Trennwand 18 endet in dem in Figur 3 gezeigten Ausführungsbeispiel an einem trennwandlosen Sumpfbereich 20, der etwa den unteren 10 bis 20 % der Böden entspricht.

Alternativ kann sich die Trennwand 18 auch bis zum Boden des Sumpfes erstrecken; vgl. Ausführungsbeispiel in Figur 4

In dem Ausführungsbeispiel der Figur 5 ist die Destillationskolonne 12 zusätzlich mit einer Sperrwand 21 versehen, welche den auf der Seite des Seitenabzugs 13 liegende Teil der Destillationskolonne 12 zum trennwandlosen Kopfbereich 19 hin verschließt.

Bei allen Ausführungsformen der Trennwandkolonnen (Figuren 3 bis 5) verläuft die Trennwand 18 im Wesentlichen vertikal durch die Destillationskolonne 12 und zwar außermittig, sodass das Verhältnis der Fläche auf der Seite des Seitenabzugs zur Fläche auf der Seite des Zulaufs des Sumpfproduktes 0.5 beträgt. Es können aber auch andere Flächenverhältnisse durch die Lage der Trennwand eingestellt werden, etwa im Bereich von 0,1 bis 0,9, bevorzugt von 0,25 bis 0,75 und ganz besonders bevorzugt von 0,4 bis 0,6.

Im trennwandlosen Kopfbereich 19 wird die Flüssigkeit in einem Verhältnis von 0,5 bis 1, vorzugsweise 0,5 bis 0,8 auf die Seite des Seitenabzugs 13 und die Seite des zuströmenden Sumpfproduktes 9 aufgeteilt.

### Bezugszeichenliste

- 1: Zusammensetzung
- 2: Quenchkolonne
- 3: wässriger Flüssigkeitsstrom
- 4: flüssige Lösung
- 5: Gasstrom
- 6: Entwässerungskolonne
- 7: Schleppmittel
- 8: Wasser/Schleppmittelazeotrop
- 9: Sumpfprodukt
- 10: Dekanter
- 11: Abwasser
- 12: Destillationskolonne
- 13: Seitenabzug
- 14: Acrylsäure
- 15: Leichtsieder (Essigsäure)
- 16: Schwersieder (Aldehyd)
- 17: Inhibitor
- 18: Trennwand
- 19: trennwandloser Kopfbereich
- 20: trennwandloser Sumpfbereich
- 21: Sperrwand

## Patentansprüche

1. Verfahren zur Abtrennung von Acrylsäure (14) aus einer Zusammensetzung (1) enthaltend Acrylsäure und zumindest ein Begleitprodukt,
a) bei welchem die Zusammensetzung (1) zunächst mit einem wässrigen Flüssigkeitsstrom (3) dergestalt kontaktiert wird, dass eine flüssige Lösung (4) enthaltend Acrylsäure, Begleitprodukt und Wasser anfällt,
b) bei welchem der flüssigen Lösung (4) ein Schleppmittel (7) zugegeben wird, welches mit Wasser der flüssigen Lösung ein heterogenes Minimalazeotrop (8) bildet,
c) bei welchem die flüssige Lösung (4) destillativ aufgetrennt wird in ein Kopfprodukt enthaltend das Minimalazeotrop (8) und in ein Sumpfprodukt (9) enthaltend Acrylsäure und Begleitprodukt,
d) bei welchem das Kopfprodukt in Wasser (3, 11) und rückführbares Schleppmittel (7) getrennt wird,
e) und bei welchem das Sumpfprodukt (9) destillativ aufgetrennt wird in Acrylsäure und Begleitprodukt,
**dadurch gekennzeichnet**,
f) dass die destillative Auftrennung des Sumpfproduktes in einer einen Seitenabzug (13) aufweisenden Destillationskolonne (12) erfolgt, aus deren Sumpf schwersiedende Bestandteile (16) des Begleitproduktes, von deren Kopf leicht siedende Bestandteile (15) des Begleitproduktes und aus deren Seitenabzug (13) die Acrylsäure (14) abgezogen wird, und dass die Acrylsäure (14) flüssig aus dem Seitenabzug (13) abgezogen wird, und dass die Destillationskolonne (12) als Trennwandkolonne mit einer sich vertikal durch die Kolonne ersteckenden Trennwand (18) ausgeführt ist.

2. Verfahren nach Anspruch 1,
**dadurch gekennzeichnet,**
**dass** sich die Trennwand (18) von einem trennwandlosen Kopfbereich (19) bis zu einem trennwandlosen Sumpfbereich (20) erstreckt.

3. Verfahren nach Anspruch 2, **gekennzeichnet durch** eine Sperrwand (21), welche den auf der Seite des Seitenabzugs (13) liegende Teil der Destillationskolonne (12) zum trennwandlosen Kopfbereich (19) hin verschließt.

4. Verfahren nach Anspruch 1,
**dadurch gekennzeichnet,**
**dass** sich die Trennwand (18) von einem trennwandlosen Kopfbereich (19) bis zum Boden des Sumpfes erstreckt.

5. Verfahren nach Anspruch 1, 2 oder 3,
**dadurch gekennzeichnet,**
**dass** sich die Trennwand (18) von einem Höhenniveau oberhalb des Niveaus des Seitenabzugs (13) bis zu einem Höhenniveau unterhalb des Niveaus des in die Destillationskolonne (12) eingeleiteten Sumpfproduktes (9) erstreckt.

6. Verfahren nach einem der vorhergehenden Ansprüche,
**dadurch gekennzeichnet,**
**dass** das Begleitprodukt mindestens einen der folgenden Stoffe enthält: Essigsäure, Wasser, eine chemische Verbindung aus der Gruppe der Aldehyde.

7. Verfahren nach einem der vorhergehenden Ansprüche,
**dadurch gekennzeichnet,**
**dass** es sich bei dem Schleppmittel um ein organisches Lösungsmittel mit einem Siedepunkt zwischen 80 und 130°C handelt, insbesondere um Toluol oder Heptan oder Cyclohexan oder Methylcyclohexan.

8. Verfahren nach einem der vorhergehenden Ansprüche,
**dadurch gekennzeichnet,**
**dass** die destillative Auftrennung der flüssigen Lösung (4) in einer Entwässerungskolonne (6) dergestalt erfolgt, dass das Sumpfprodukt (9) nahezu wasserfrei ist und die Konzentration des Schleppmittels (7) in dem Sumpfprodukt (9) 1 bis 15, bevorzugt 8 bis 12 Gew.-% beträgt.

## Claims

1. Process for separating acrylic acid (14) from a composition (1) containing acrylic acid and at least one accompanying product,
a) in which the composition (1) is firstly contacted with an aqueous liquid stream (3) in such a way that a liquid solution (4) containing acrylic acid, accompanying product and water is obtained,
b) in which an entrainer (7) which forms a heterogeneous minimum azeotrope (8) with the water of the liquid solution (4) is added to the liquid solution,
c) in which the liquid solution (4) is separated by distillation into an overhead product containing the minimum azeotrope (8) and a bottom product (9) containing acrylic acid and accompanying product,
d) in which the overhead product is separated into water (3, 11) and recirculatable entrainer (7)
e) and in which the bottom product (9) is separated by distillation into acrylic acid and accompanying product,
**characterized in that**
f) the fractional distillation of the bottom product is carried out in a distillation column (12) which has a side offtake (13) and from the bottom of which high-boiling constituents (16) of the accompanying product are taken off, from the top of which low-boiling constituents (15) of the accompanying product are taken off and from the side offtake (13) of which the acrylic acid (14) is taken off and, **in that** the acrylic acid (14) is taken off in liquid form from the side offtake (13) and **in that** the distillation column (12) is configured as a dividing wall column having a dividing wall (18) extending vertically through the column.

2. Process according to Claim 1, **characterized in that** the dividing wall (18) extends from a dividing wall-free top region (19) to a dividing wall-free bottom region (20).

3. Process according to Claim 2, **characterized by** a barrier wall (21) which closes off the part of the distillation column (12) located on the side of the side offtake (13) from the dividing wall-free top region (19).

4. Process according to Claim 1, **characterized in that** the dividing wall (18) extends from a dividing wall-free top region (19) to the floor of the bottom.

5. Process according to Claim 1, 2 or 3, **characterized in that** the dividing wall (18) extends from a height level above the level of the side offtake (13) to a height level below the level of the bottom product (9) introduced into the distillation column (12).

6. Process according to any of the preceding claims, **characterized in that** the accompanying product contains at least one of the following substances:
acetic acid, water, a chemical compound from the group consisting of aldehydes.

7. Process according to any of the preceding claims, **characterized in that** the entrainer is an organic solvent having a boiling point in the range from 80 to 130°C, in particular toluene or heptane or cyclohexane or methylcyclohexane.

8. Process according to any of the preceding claims, **characterized in that** the fractional distillation of the liquid solution (4) in a dewatering column (6) is carried out in such a way that the bottom product (9) is virtually water-free and the concentration of the entrainer (7) in the bottom product (9) is from 1 to 15% by weight, preferably from 8 to 12% by weight.

## Revendications

1. Procédé pour la séparation d'acide acrylique (14) d'une composition (1) contenant de l'acide acrylique et au moins un produit secondaire,
a) dans lequel la composition (1) est d'abord mise en contact avec un flux (3) liquide aqueux de manière telle qu'une solution liquide (4) contenant de l'acide acrylique, du produit secondaire et de l'eau est obtenue,
b) dans lequel la solution liquide (4) est additionnée d'un agent d'entraînement (7) qui forme un azéotrope minimal hétérogène (8) avec l'eau de la solution liquide,
c) dans lequel la solution liquide (4) est séparée par distillation en un produit de tête contenant l'azéotrope minimal (8) et en un résidu (9) contenant de l'acide acrylique et du produit secondaire,
d) dans lequel le produit de tête est séparé en eau (3, 11) et en agent d'entraînement (7) recyclable,
e) et dans lequel le résidu (9) est séparé par distillation en acide acrylique et en produit secondaire, **caractérisé**
f) **en ce que** la séparation par distillation du résidu est réalisée dans une colonne de distillation (12), présentant un soutirage latéral (13), du fond de laquelle on soutire les constituants (16) à point d'ébullition élevé du produit secondaire, de la tête de laquelle on soutire les constituants (15) à bas point d'ébullition du produit secondaire et du soutirage latéral (13) de laquelle on soutire l'acide acrylique (14), et l'acide acrylique (14) est soutiré sous forme liquide du soutirage latéral (13) et la colonne de distillation (12) est conçue comme une colonne à paroi de séparation présentant une paroi de séparation (18) s'étendant verticalement à travers la colonne.

2. Procédé selon la revendication 1, **caractérisé en ce que** la paroi de séparation (18) s'étend d'une zone de tête (19) sans paroi de séparation jusqu'à une zone de fond (20) sans paroi de séparation.

3. Procédé selon la revendication 2, **caractérisé par** une paroi de blocage (21), qui ferme la partie de la colonne de distillation (12) se trouvant du côté du soutirage latéral (13) vers la zone de tête (19) sans paroi de séparation.

4. Procédé selon la revendication 1, **caractérisé en ce que** la paroi de séparation (18) s'étend d'une zone de tête (19) sans paroi de séparation jusque dans le bas du fond.

5. Procédé selon la revendication 1, 2 ou 3, **caractérisé en ce que** la paroi de séparation (18) s'étend d'un niveau de hauteur au-dessus du niveau du soutirage latéral (13) jusqu'à un niveau de hauteur sous le niveau du résidu (9) introduit dans la colonne de distillation (12).

6. Procédé selon l'une quelconque des revendications précédentes, **caractérisé en ce que** le produit secondaire contient au moins une des substances suivantes : acide acétique, eau, un composé chimique du groupe d'aldéhydes.

7. Procédé selon l'une quelconque des revendications précédentes, **caractérisé en ce qu'**il s'agit, pour l'agent d'entraînement, d'un solvant organique présentant un point d'ébullition entre 80 et 130°C, en particulier de toluène ou d'heptane ou de cyclohexane ou de méthylcyclohexane.

8. Procédé selon l'une quelconque des revendications précédentes, **caractérisé en ce que** la séparation par distillation de la solution liquide (4) a lieu dans une colonne de déshydratation (6), de manière telle que le résidu (9) est pratiquement exempt d'eau et la concentration en agent d'entraînement (7) dans le résidu (9) est de 1 à 15, de préférence de 8 à 12% en poids.
